# EUROPEAN PATENT APPLICATION

(11) **EP 4 523 645 A1**
(43) Date of publication of application: **19.03.2025**
(21) Application number: 23197363.7
(22) Date of filing: 14.09.2023
(51) Int. Cl.: A61B 34/20

(54) **SURGICAL TUBE ASSEMBLY AND SURGICAL INSTRUMENT COMPRISING THE TUBE ASSEMBLY**

(71) Applicant: Stryker European Operations Limited, Carrigtwohill, Co. Cork T45 HX08 (IE)
(72) Inventor: DINGERT, Michael, 13053 Berlin (DE); REUTTER, Andreas, 10437 Berlin (DE); GLANERT, Maresa, 10318 Berlin (DE)
(74) Representative: Röthinger, Rainer

(57) **Abstract**

A surgical tube assembly comprising an outer tube and an inner tube is presented. The inner tube has a uniform wall thickness. In a first section of the surgical tube assembly, a first circumferential portion of the inner tube is supported at the outer tube and at least one first space configured to accommodate a sensor element is defined between at least one second circumferential portion of the inner tube and the outer tube. Further presented are a surgical suction instrument and a surgical endoscope comprising the surgical tube assembly.

## Description

### Technical Field

The present disclosure relates to a surgical tube assembly. The surgical tube assembly presented herein may be comprised by a surgical instrument such as a surgical suction instrument or a surgical endoscope.

### Background

Surgical instruments such as surgical suction instruments or surgical endoscopes are often designed to a have a tube assembly. One end of the tube assembly is typically connected to a handle portion of the surgical instrument and the other end is free to enter a patient's body to perform a surgical procedure or for other purposes.

Having entered the patient's body with the free end of the tube assembly, a surgeon often can no longer see the portion of the tube assembly located in the body of the patient at its current location within the body of the patient. In order to still allow a safe and reliable navigation of the surgical instrument, image-guided surgery (IGS) can be used.

IGS is a technique which obtains a real-time correlation of a location of at least a portion of an instrument that has been inserted into a patient's body to a pre- or intra-operatively obtained image of a patient anatomy (e.g., a computer tomography, CT, scan, an X-ray image, a magnetic resonance imaging, MRI, scan, etc.), such that the current location of said portion of the instrument can be superimposed on the image of the patient anatomy. To this end, the image is displayed to the surgeon together with an indicator (e.g., crosshairs, a colored dot, etc.) highlighting the real-time position of the surgical instrument relative to the anatomical structures shown in the image. In this manner, the surgeon is able to infer the precise position of the instrument relative to the patient anatomy even if the surgeon is unable to directly see the instrument itself at its current location within the body of the patient.

IGS relies on tracking techniques for a real-time tracking of surgical instruments in an operating room relative to a patient. To this end, the instruments are equipped with tracking elements. In electromagnetic and similar tracking scenarios, the instruments are equipped with one or more sensor elements (e.g., one or more coils configured to measure externally generated electromagnetic fields). During a surgical procedure, the sensor elements send signals to circuitry that is configured to evaluate the signals and determine the current position of each surgical instrument.

EP 3 409 219 A1 describes a tube assembly of a suction instrument. The tube assembly includes an external sheath, an interior suction tube, a mounted sensor assembly, and a distal cap. The external sheath defines a hollow interior that extends from an open proximal end to an open distal end. The hollow interior is dimensioned to house a portion of the interior suction tube as well as a portion of a communication wire extending within and along a guided path defined by the interior suction tube and coupled to an annular sensor element by means of coupling wires.

EP 3 530 218 A1 describes a tube having two straight portions and a bent portion interposed between the two straight portions. An elastic guidewire channel is secured along the length of the bent portion and along at least a part of each straight portion. A guidewire with a rigid end portion and a sensor element located therein is accommodated in the elastic guidewire channel.

### Summary

There is a need for a surgical tube assembly that accommodates a sensor element in an efficient manner.

A first aspect of the present disclosure relates to a surgical tube assembly comprising an outer tube and an inner tube, wherein the inner tube has a uniform wall thickness and wherein, in a first section of the surgical tube assembly, a first circumferential portion of the inner tube is supported at the outer tube and at least one first space configured to accommodate a sensor element is defined between at least one second circumferential portion of the inner tube and the outer tube.

The surgical tube assembly may have a linear configuration or comprise one, two or more bends. In a bent configuration, the surgical tube assembly may generally have an L-shape, a C-shape or a Z-shape.

At least one supporting element (e.g. a spacer) may be arranged between the first circumferential portion of the inner tube that is supported at the outer tube and the outer tube. The supporting element may be configured to attach the first circumferential portion of the inner tube to the outer tube. Alternatively, the first circumferential portion of the inner tube may directly be attached to the outer tube (e.g. by a friction fit or by gluing).

In certain implementations, the inner tube may have a non-uniform wall thickness. As an example, the wall of the inner tube may comprise one or more grooves or protrusions on at least one of an inner and an outer surface thereof.

The first space configured to accommodate the sensor element may be defined between an outer surface of the second circumferential portion of the inner tube and an inner surface of the outer tube. The outer tube may have a continuously curved outer surface (e.g. it may be an elliptical or a circular cross-section). The first section of the surgical tube assembly may be plane-symmetrical to one plane or to two perpendicular planes.

In the first section, the second circumferential portion may be flat (e.g. planar). It may be manufactured by a forming method (e.g. by further forming an elliptical cylinder or a circular cylinder). The second circumferential portion may be more flexible in its circumferentially central region than in its circumferentially outer regions, which are closer to the first circumferential portion.

In the first section, a transition from an outer surface of the second circumferential portion to an outer surface of the first circumferential portion may be curved. The curvature may be manufactured by a forming method (e.g. by further forming an elliptical cylinder or a circular cylinder).

In the first section, an outer surface of the first circumferential portion may abut an inner surface of the outer tube. The outer surface of the first circumferential portion may completely or partially abut the inner surface of the outer tube. The abutment may be such that the inner tube is prevented from movement within the outer tube (e.g., in a direction that is orthogonal to an axial direction, i.e. that is orthogonal to a center line of the outer tube).

The first circumferential portion may extend circumferentially a first angle a about a centre line of the outer tube in the first section and the second circumferential portion may extend circumferentially a second angle β about the centre line of the outer tube. The angle α may be in the range from 340° to 180°, e.g. in the range from 315° to 180°. The angle β may be in the range from 20° to 180°, e.g. in the range from 45° to 180°. More specifically, the angle α may be 250° ± 5° and the angle β may be 110° ± 5°.

In some implementations, the first circumferential portion may be divided into multiple first circumferential portions, which are circumferentially spaced apart from each other, and the second circumferential portion may be divided into multiple second circumferential portions, which are circumferentially distanced from each other. The number, e.g. two, of first circumferential portions may equal the number, e.g. two, of second circumferential portions. The first circumferential portions may each extend circumferentially an angle α about the centre line of the outer tube and the second circumferential portions may each extend circumferentially a second angle β about the centre line of the outer tube. The total sum of the angles α may be in the range from 320° to 1°, e.g. in the range from 180° to 1°. The total sum of the angles β may be in the range from 40° to 359°, e.g. in the range from 180° to 359°. More specifically, the total sum of the angles α may be 140° ± 5° and the total sum of the angles β may be 220° ± 5°.

In the first section, there may be two second circumferential portions on opposite sides of a plane longitudinally intersecting the outer tube, and, consequently, two first spaces. In some variants, each of the two first spaces is configured to accommodate, or accommodates, a sensor element. In other variants, only one of the two first spaces is configured to accommodate, or accommodates, a sensor element, while the other first space is configured to accommodate, or accommodates, a different component (e.g. accommodates a light source). The first section of the surgical tube assembly may be plane-symmetrical to two planes that are orthogonal to each other.

In the first section, the inner tube may have a D-shaped cross-section. In other implementations, the inner tube may have a cross-section resembling an 8, defining two closed or interconnected first spaces. Alternatively or additionally, the wall thickness of the inner tube may be less than the wall thickness of the outer tube, e.g. in the first section. In a cross-section of the first section, an area occupied by a wall of the inner tube may be less than an area enclosed by a wall of the inner tube.

In a second section of the surgical tube assembly that is spaced apart from the first section along an extension of the surgical tube assembly, the first circumferential portion may be larger and the second circumferential portion may be smaller than in the first section. This configuration may lead to a second space that is narrower than the first space, wherein the first space is in communication with the second space. The second space may be configured to accommodate electrical wiring (e.g. for electrically contacting the sensor element). So, the second space may be too narrow for accommodating the sensor element located in the first space and create a stop for the sensor element in an axial direction. The second space may be open at a first end of the surgical tube assembly. The first end of the surgical tube assembly may face towards or be surrounded by a handle portion of a surgical instrument comprising the surgical tube assembly.

In the second section, the inner tube may have a D-shaped cross-section. In other implementations, the inner tube may have a cross-section resembling an 8, defining two closed or interconnected second spaces.

The surgical tube assembly may comprise the sensor element, which is located in the first space. Of course, in case there are multiple second circumferential portions and, therefore, multiple first spaces, a sensor element can be provided in each of the multiple first spaces. The sensor element may be an electromagnetic sensor (e.g. a coil) for electromagnetic tracking. The sensor element may be fixed to at least the second circumferential portion. The sensor element may be permanently bonded to the second circumferential portion, for example by glue.

The first space may be arranged in a region of a second end of the surgical tube assembly and closed at the second end. The second end may be facing a patient during a surgical intervention. The surgical tube assembly may further comprise a plug closing the first space at the second end. The plug may abut the sensor element, for example in the assembled state of the surgical tube assembly. Further, the plug may leave an axial end or an axial end portion of the inner tube exposed.

One variant of the plug closing the first space may have a free dome-shaped end. This plug may have an opposite wedge-shaped end inserted into the first space. The plug may axially abut only the outer tube and be axially spaced apart from the inner tube. The plug may be plane-symmetrical to a plane.

Another variant of the plug may have a free pill-shaped end. This plug may have an opposite chamfered plug-shaped end inserted into the first space. The plug may axially abut only the outer tube and be axially spaced apart from the inner tube.

A further variant of the plug may have a U-shape with a hole in the base of the U-shape for the inner tube. So, the base of the U-shaped plug may form a free end of the plug and the walls of the U-shaped plug may form an opposite end of the plug. The entire plug may be referred to as olive-shaped with a through-hole that at a free end portion of the plug fits on the inner tube and at the opposite end portion of the plug fits on the outer tube. The plug may axially only abut the outer tube and circumferentially enclose an end part of the outer tube as well as an end part of the inner tube. The plug may have an axial end, which is axially flush with an end of the inner tube, in the assembled state of the third surgical tube assembly.

A still further variant of the plug may be shaped as a hollow cylinder with a uniform through-hole. A free end of the plug may circumferentially only enclose an end part of the inner tube and may axially only about the outer tube. The plug may have the same outer shape and outer dimensions as the outer tube, such that the plug continues the outer shape of the outer tube. An opposite end may be a wedge-shaped portion of the plug that is inserted into the first space such that it is sandwiched between the second circumferential portion of the inner tube and the outer tube.

Another variant of the plug may correspond to the previous plug but be plane-symmetrical to an additional plane, e.g. a first plane.

A further aspect relates to a surgical instrument configured as a surgical suction instrument comprising the surgical tube assembly presented herein, wherein the inner tube defines a suction channel.

A still further aspect relates to a surgical instrument configured as a surgical endoscope comprising the surgical tube assembly, wherein the inner tube houses one or more endoscopic components.

Another solution is a surgical tube assembly comprising an outer tube and an inner tube located within the outer tube, wherein, in a first section of the surgical tube assembly, the inner tube has a D-shaped cross-section, a first circumferential portion of the inner tube is supported at the outer tube, and a first space configured to accommodate a sensor element is defined between a second circumferential portion of the inner tube and the outer tube. In other implementations, the inner tube may have a cross-section resembling an 8, defining two closed or interconnected first spaces.

The latter solution may comprise an inner tube that has a uniform wall thickness. Alternatively or additionally, the latter solution may be further defined by any of the above mentioned provisions.

Another solution is a method for manufacturing one of the above surgical tube assemblies, the surgical suction instrument, or the surgical endoscope, wherein the method comprises the following steps: inserting the inner tube axially into the outer tube and fixing the inner tube to the outer tube. The inner tube and the outer tube may have a linear configuration when inserting the inner tube into the outer tube. The inner tube may then be fixed to the outer tube by bending the linear tubes after the inner tube has been inserted into the outer tube (which can result in a friction fit between the tubes).

The inner tube may have been formed as needed prior to inserting it into the outer tube. Before inserting the inner tube into the outer tube, the sensor element may be fixed to the second circumferential portion of the inner tube, for example by means of glue. Alternatively, after inserting the inner tube, the sensor element may be inserted and sandwiched between the second circumferential portion of the inner tube and the outer tube. Then, the sensor element may be pushed (e.g. by means of the plug) further towards the inside of the outer tube (e.g. until the plug abuts an axial end of the outer tube). In a further step, the plug may be fixed to the outer tube, e.g. by means of glue.

A step or structural provision may require that the sensor element is inserted into the outer tube such that the entire axial length of the sensor element is circumscribed by the outer tube. A step or structural provision may require that the sensor element engages the outer tube in a form-fit, which avoids a movement of the sensor element in the circumferential direction around the center line of the outer tube, and engages the inner tube and the outer tube in a friction fit, which avoids a movement of the sensor element in an axial direction, i.e. in a direction of the center line of the outer tube.

### Brief Description of the Drawings

The foregoing summary, as well as the following detailed description, will be better understood when read in conjunction with the appended drawings. For the purpose of illustrating the present disclosure, preferred embodiments are shown in the drawings. It should be understood, however, that the invention is not limited to the specific embodiments disclosed in the drawings, and reference is made to the claims for that purpose. In the drawings:
- Fig. 1: is a perspective view of a surgical instrument with a handle portion and a portion comprising a surgical tube assembly;
- Fig. 2: is a partially exploded perspective view of the surgical instrument of Fig. 1, and in particular of the surgical tube assembly;
- Fig. 3: is another partially exploded perspective view of a free end of a first surgical tube assembly with an outer tube and an inner tube located within the outer tube, wherein a first plane and a second plane are shown intersecting each other along a center line of the outer tube;
- Fig. 4: is a longitudinal cross-section view through the second plane of the assembled surgical tube assembly of Fig. 3;
- Fig. 5: is a longitudinal cross-section view through the second plane of a second surgical tube assembly;
- Fig. 6: is a longitudinal cross-section view through the second plane of a third surgical tube assembly;
- Fig. 7: is a longitudinal cross-section view through the second plane of a fourth surgical tube assembly;
- Fig. 8: shows the cross-section A-A of Fig. 7;
- Fig. 9: shows the cross-section B-B of Fig. 7;
- Fig. 10: is a longitudinal cross-section view through the second plane of a fifth surgical tube assembly; and
- Fig. 11: shows the cross-section C-C of Fig. 10.

### Detailed Description

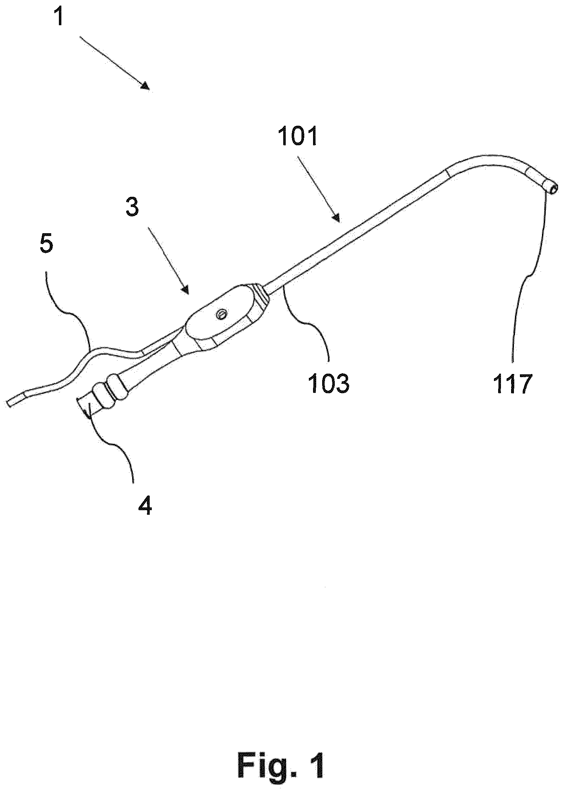

Fig. 1 is a perspective view of a surgical instrument 1 with a handle portion 3 and a portion comprising an L-shaped surgical tube assembly 101. The surgical instrument 1 is configured as a surgical suction instrument that can be connected to a suction source (not shown) via a suction port 4 at the handle portion 3. However, the surgical instrument 1 can alternatively be configured as a surgical endoscope or any other instrument.

The surgical instrument 1 is configured to be tracked for IGS purposes. In more detail, the surgical instrument 1 is configured for electromagnetic tracking. To this end, the surgical instrument 1 comprises a sensor element (reference numeral 113, not shown in Fig. 1) at its free end opposite the handle portion 3. The sensor element is electrically connected to a cable 5 extending from the handle portion 3. The cable 5 is configured to be connected to a measurement unit (not shown) of an electromagnetic tracking system.

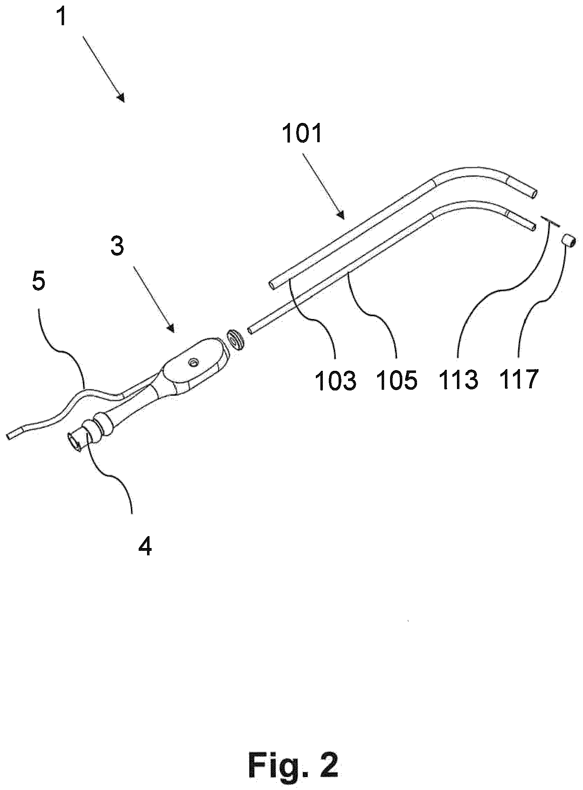

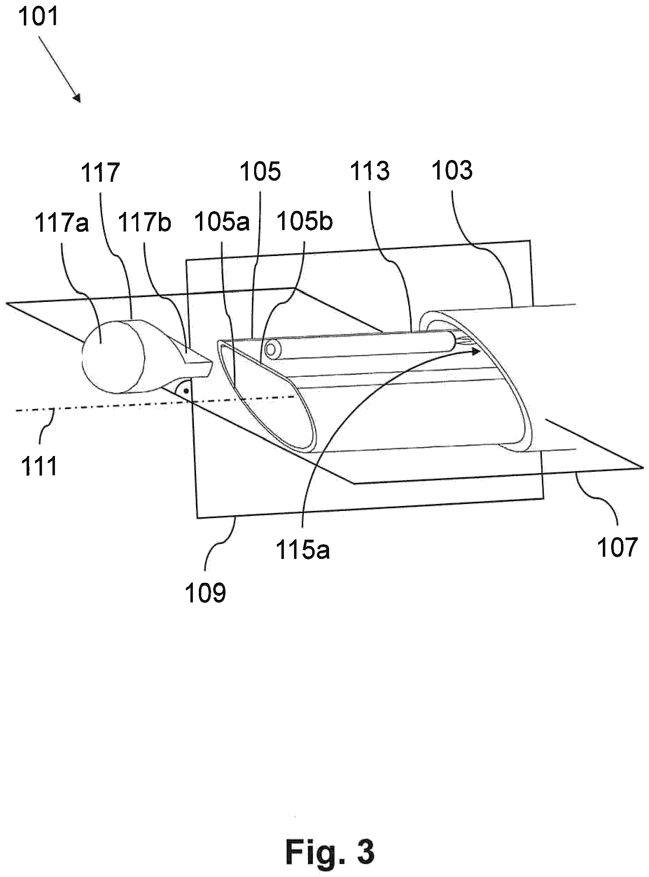

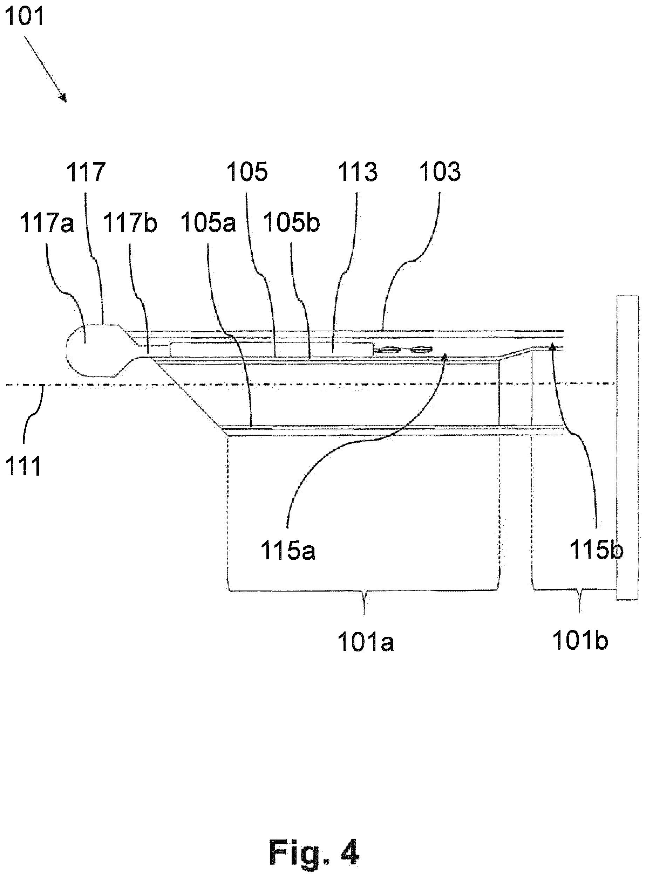

Fig. 2 is a partially exploded perspective view of the surgical instrument 1 of Fig. 1, and in particular of the surgical tube assembly 101. Fig. 3 is another exploded perspective view of a free end of the surgical tube assembly 101. As can be gathered from Fig. 2 and Fig. 3, the surgical tube assembly 101 comprises an outer tube 103 (see also Fig. 1) and an inner tube 105 located within the outer tube 103. In Fig. 3, a first plane 107 and a second plane 109 arranged perpendicular to the first plane 107 are shown intersecting each other along a center line 111 of the outer tube 103. Fig. 4 is a longitudinal cross-section view through the second plane 109 of the assembled surgical tube assembly 101 of Fig. 3.

As shown in Fig. 3, the inner tube 105 in this variant has a uniform wall thickness, and the same applies to the outer tube 103. The wall thickness of the inner tube 105 ranges between 0.01mm and 5mm. The wall thickness of the outer tube 103 ranges between 0.01mm und 10mm. In other instances, one or both of the inner tube 105 and the outer tube 103 may have a non-uniform wall thickness. As an example, the wall of the inner tube 105 may comprise one or more grooves milled into at least one of an inner and an outer surface thereof.

In a first section 101a of the surgical tube assembly 101 shown in Fig. 4, a first circumferential portion 105a of the inner tube 105 is directly supported at the outer tube 103. In other variants, one or more supporting elements can be provided therebetween.

A sensor element 113 (see also Fig. 2) is accommodated in a first space 115a defined between an outer surface of a second circumferential portion 105b of the inner tube 105 and an inner surface of the outer tube 103. The sensor element 113 has a cylindrical shape. The sensor element 113 can be configured as a sensor coil for electromagnetic tracking purposes. In such a configuration, the sensor element 113 is adapted to sense a locally variable electromagnetic field generated by an electromagnetic field generator (not shown) of the electromagnetic tracking system. The sensor element 113 is connected to electrical wiring that passes through a length of the tube assembly 101 and a section of the handle portion 3 to leave the handle portion 3 via the cable 5 (see Fig. 1).

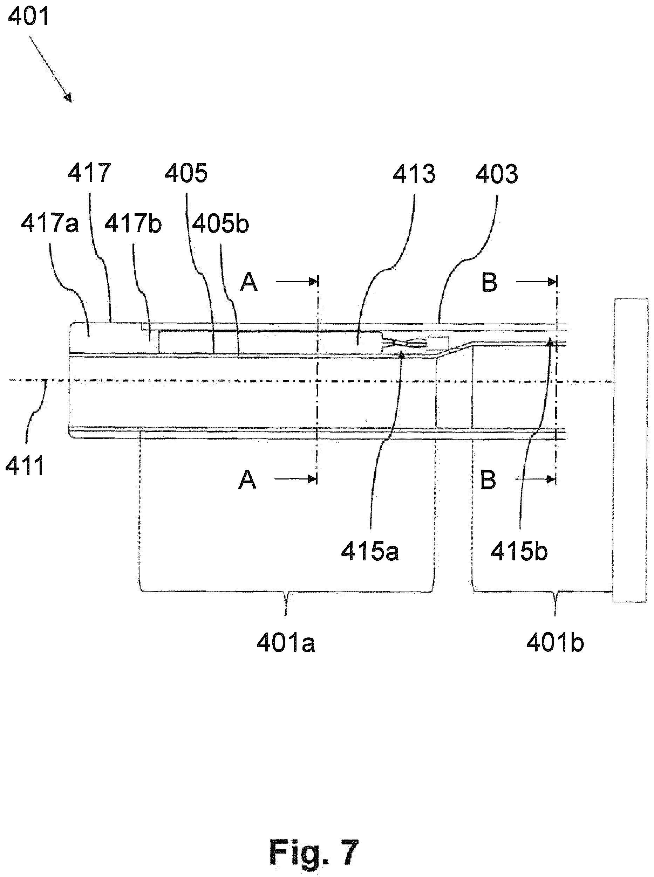

The sensor element 113 is located in the first space 115a and either fixed to the second circumferential portion 105a of the inner tube 105, e.g. by a permanent bond such as by means of glue as is the case here, or sandwiched between the second circumferential portion 105a of the inner tube 105 and the outer tube 103 as shown in Fig. 7 to Fig. 11. The outer tube 103 may have a continuously curved outer surface (e.g. it may have an elliptical or a circular cross-section as shown in Fig. 3, 8 and 11).

Fig. 3 further shows that here, in the first section 101a as illustrated in Fig. 4, the second circumferential portion 105b is flat, giving rise to a substantially D-shaped cross-section of the inner tube 105. The flat portion 105b may be manufactured by a forming method (e.g. by further forming an elliptical cylinder or a circular cylinder). The inner tube 105 having a uniform wall thickness and the second circumferential portion 105b being flat causes the second circumferential portion 105b to be more flexible in its circumferentially central region than in its circumferentially outer regions, which are closer to the first circumferential portion 105a. This helps in sandwiching the sensor element 113 between the second circumferential portion 105b of the inner tube 105 and the outer tube 103.

Fig. 3 also shows that a transition from an outer surface of the second circumferential portion 105b to an outer surface of the first circumferential portion 105a is curved. Thereby, damage to the inner surface of the outer tube 103 can be avoided. The curvature may be manufactured by a forming method (e.g. by further forming an elliptical cylinder or a circular cylinder).

Fig. 3 further shows that an outer surface of the first circumferential portion 105a abuts an inner surface of the outer tube 103. The outer surface of the first circumferential portion 105a may completely or partially abut the inner surface of the outer tube 103. The abutment may be such that the inner tube 105 is prevented from movement within the outer tube 103 in a direction that is orthogonal to the center line 111 of the outer tube 103 (e.g., due to a friction fit between the two tubes 103, 105 or a local deformation along an extension of the two tubes 103, 105).

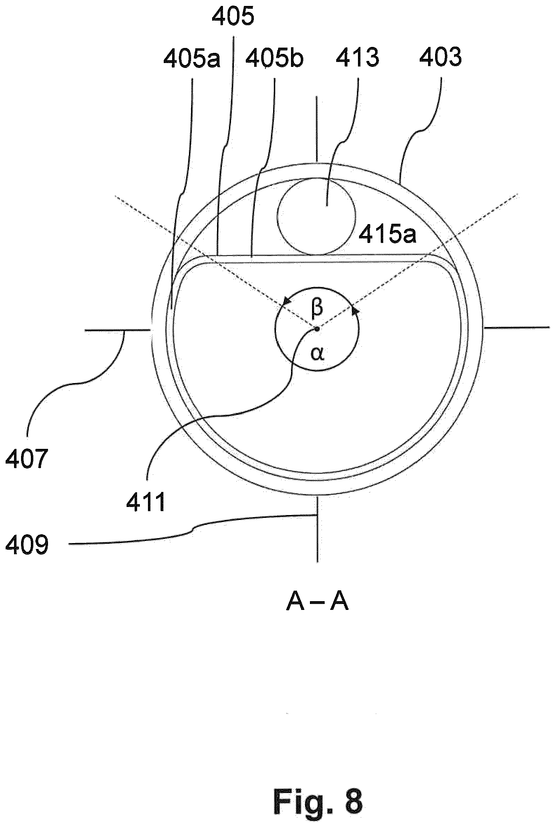

Fig. 3 further shows that in a cross-section of the first section 101a, an area occupied by a wall of the inner tube 105 is less than an area enclosed by a wall of the inner tube 105 - see also Fig. 8 for comparison. Here, in the first section 101a, the wall thickness of the inner tube 105 is less than the wall thickness of the outer tube 103. In other implementations, the inner tube 105 may have a cross-section resembling an 8, defining two closed or interconnected first spaces 115a.

In a second section 101b of the surgical tube assembly 101 that is only partially shown in Fig. 4 and spaced apart from the first section 101a along an extension of the surgical tube assembly 101, the first circumferential portion 105a is larger and the second circumferential portion 105b is smaller than in the first section 101a. This configuration may lead to a second space 115b that is narrower in a direction of the second plane 109 than the first space 115a. The first space 115a is in communication with the second space 115b. In some variants, the second space 115b is too narrow in a direction of the second plane 109 for accommodating the sensor element 113 located in the first space 115a and, thereby, creates a stop for the sensor element 113 in an axial direction, i.e. in a direction of the center line 111 of the outer tube 103. The inner tube 105 may have a circular cross-section in the second section 101b of the surgical tube assembly 101.

While the second space 115b is open at a first end of the surgical tube assembly 101, the first space 115a is arranged in a region of a second end of the surgical tube assembly 101 and closed at the second end, wherein the second end of the surgical tube assembly 101 is opposite the first end of the surgical tube assembly 101. The second end may be facing a patient during a surgical intervention. The second space 115b is configured to accommodate electrical wiring (e.g. for electrically contacting the sensor element 113). The electrical wiring is shown by the two wires extending to the right from the sensor element 113 in Fig. 4. The first end of the surgical tube assembly 101 may face towards or be surrounded by the handle portion 3 of the surgical instrument 1 comprising the surgical tube assembly 101. Here, the second end of the outer tube 103 and the second end of the inner tube 105 are chamfered, such that the respective ends are flush, when the inner tube 105 is fully inserted into the outer tube 103(i.e. in the assembled state of the surgical tube assembly 101 shown in Fig. 4).

A plug 117 (see also Fig. 1 and Fig. 2) closes the first space 115a at the second end, i.e. a first axial end, of the surgical tube assembly 101. Here, the plug 117 abuts the sensor element 113. Further, the plug 117 leaves an axial end portion of the inner tube 105 exposed. Also, here, the plug 117 has a free dome-shaped end 117a and an opposite end 117b (e.g. the here shown wedge-shaped end 117b) inserted into the first space 115a. The plug 117 axially abuts only the outer tube 103 and is axially spaced apart from the inner tube 105. The plug 117 and the first section 101a of the surgical tube assembly 101 are plane-symmetrical to a plane, i.e. the second plane 109.

In a surgical suction instrument, the inner tube 105 defines a suction channel. In a surgical endoscope, the inner tube 105 houses one or more endoscopic components, e.g. optical elements. The surgical tube assembly 101 may have a linear configuration or comprise one, two or more bends. In a bend configuration, the surgical tube assembly 101 may generally have an L-shape, a C-shape or a Z-shape.

Fig. 3 further illustrates a method for manufacturing the surgical tube assembly 101. The method comprises inserting the inner tube 105 axially into the outer tube 103 and fixing the inner tube 105 to the outer tube 103. At least one supporting element (e.g. a spacer, not shown) may be arranged between the first circumferential portion 105a of the inner tube 105 supported at the outer tube 103 and the outer tube. The supporting element may be configured to attach the first circumferential portion 105a of the inner tube 105 to the outer tube 103. Alternatively or in addition, as shown here, the first circumferential portion 105a of the inner tube 105 may be supported at the outer tube 103 by directly attaching, for example by establishing a friction fit between and/or by gluing, the first circumferential portion 105a of the inner tube 105 to the outer tube 103. In some variants, the friction fit is established by bending an initially straight, or linear, configuration of the tube assembly 101.

Before inserting the inner tube 105, the sensor element 113 (with the electrical wiring attached) may be fixed to the second circumferential portion 105b of the inner tube 105, for example by means of glue. In this case, the inner tube 105 may have been pre-formed to have the D-shaped cross section at its free end opposite the handle portion 3 to receive the sensor element 113. In further scenarios, after inserting the inner tube 105, the sensor element 113 may be inserted and sandwiched between the second circumferential portion 105b of the inner tube 105 and the outer tube 103. Then, the sensor element 113 is pushed by means of the plug 117, or otherwise, further towards the inside of the outer tube 103, for example until the plug 117 abuts an axial end of the outer tube 103. In a further step, the plug 117 may be fixed to the outer tube 103, e.g. by means of glue. A step or structural provision may require that the sensor element 113 is inserted into the outer tube 103 such that the entire axial length of the sensor element 113 is circumscribed by the outer tube 103. A step or structural provision may require that the sensor element 113 engages the outer tube 103 in a form-fit, which avoids a movement of the sensor element 113 in the circumferential direction around the center line 111 of the outer tube 103, and engages the inner tube 105 and the outer tube 103 in a friction fit, which avoids a movement of the sensor element 113 in an axial direction, i.e. in a direction of the center line 111 of the outer tube 103.

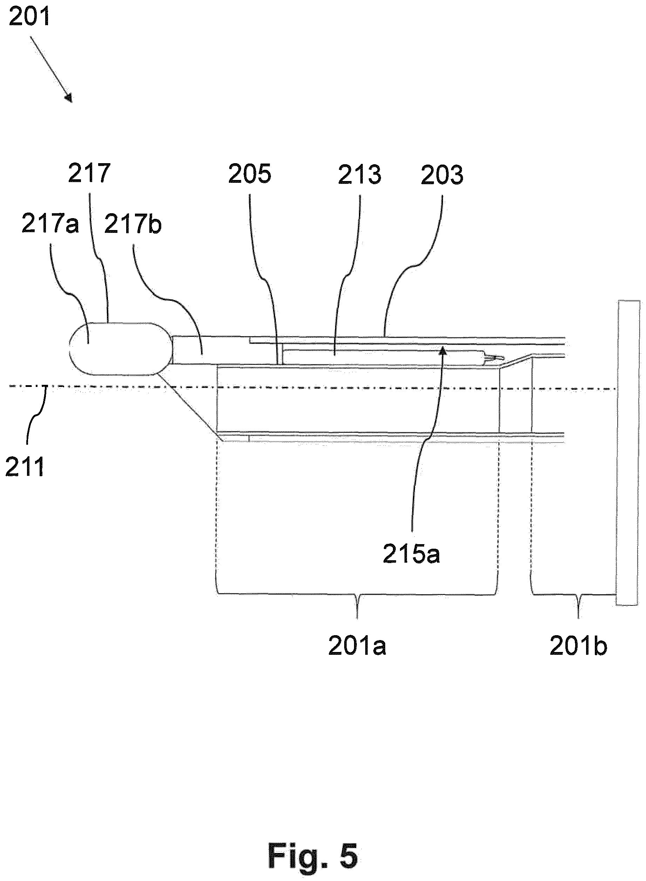

Fig. 5 is a longitudinal cross-section view through the second plane of a second surgical tube assembly 201.

The second surgical tube assembly 201 differs from the first surgical tube assembly 101 of Fig. 1 to 4 in the plug 217, which closes the first space 215a. The plug 217 of the second surgical tube assembly 201 has a free pill-shaped end 217a and an opposite end 217b (e.g. the here shown chamfered plug-shaped end 217b) inserted into the first space 215a. The plug 217 axially only abuts the outer tube 203 and is axially spaced apart from the inner tube 205.

Further, here, the second end of the outer tube 203 and the second end of the inner tube 205 each extend along a plane that is orthogonal to the center line 211 of the outer tube 203, wherein both planes are parallel to each other and the inner tube 205 protrudes from the outer tube 203.

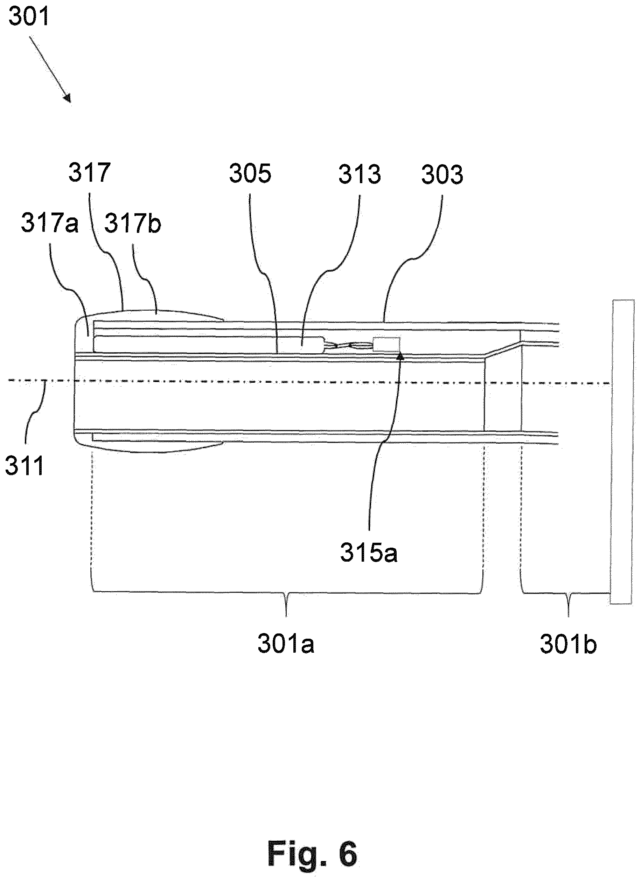

Fig. 6 is a longitudinal cross-section view through the second plane of a third surgical tube assembly 301.

The third surgical tube assembly 301 differs from the second surgical tube assembly 201 in the plug 317, which closes the first space 315a. The plug 317 has a U-shape with a hole in the base of the U-shape for the inner tube 305. So, the base of the U-shaped plug 317 forms a free end of the plug 317 and the walls of the U-shaped plug 317 form an opposite end of the plug 317. The entire plug 317 may be referred to as olive-shaped with a through-hole that at a free end portion 317a of the plug 317 fits on the inner tube 305 and at the opposite end portion 317b of the plug 317 fits on the outer tube 303. The plug 317 axially only abuts the outer tube 303 and circumferentially encloses an end part of the outer tube 303 as well as an end part of the inner tube 305. The plug 317 has an axial end, which is axially flush with an end of the inner tube 305, in the assembled state of the third surgical tube assembly 301.

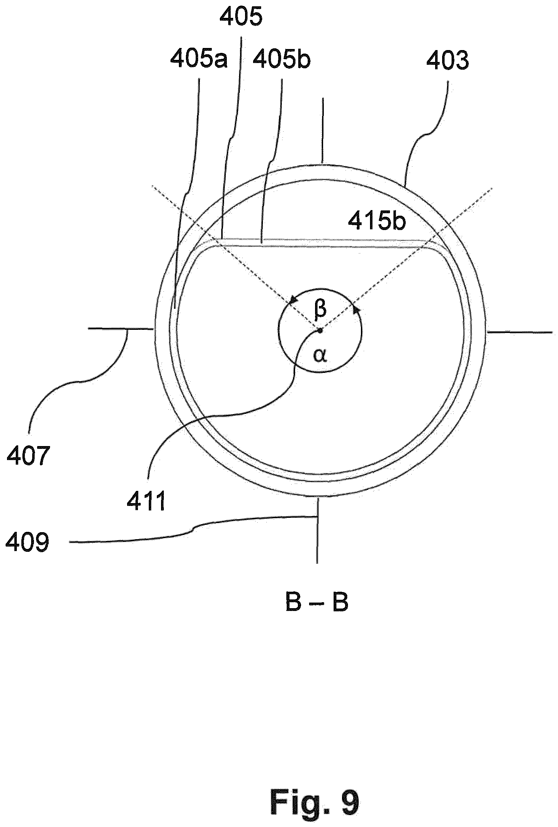

Fig. 7 is a longitudinal cross-section view through the second plane of a fourth surgical tube assembly 401. Fig. 8 shows the cross-section A-A of Fig. 7 with the first space 415a. Fig. 9 shows the cross-section B-B of Fig. 7 with the second space 415b. It will be appreciated that the previously discussed surgical tube assemblies will have cross-sections essentially similar to those depicted in Figs. 7 and 8.

The fourth surgical tube assembly 401 differs from the third surgical tube assembly 301 in the plug 417, which closes the first space 415a. The plug 417 is shaped as a hollow cylinder with a uniform through-hole. A free end 417a of the plug 417 circumferentially only encloses an end part of the inner tube 405 and axially only abuts the outer tube 403. The plug 417 has the same outer shape and outer dimensions as the outer tube 403, such that the plug 417 continues the outer shape of the outer tube 403. An opposite end 417b is a wedge-shaped portion of the plug 417 that is inserted into the first space 415a such that it is sandwiched between the second circumferential portion 405b of the inner tube 405 and the outer tube 403.

Further, here, the inner tube 405 protrudes more from the outer tube 403 compared to the third surgical tube assembly 301. The sensor element 413 is sandwiched between the second circumferential portion 405b of the inner tube 405 and the outer tube 403.

The first circumferential portion 405a extends circumferentially a first angle α about a centre line 411 of the outer tube 403 in the first section 401a and the second circumferential portion 405b extends circumferentially a second angle β about the centre line 411 of the outer tube 403. The angle α may be in the range from 340° to 180°, e.g. in the range from 315° to 180°. The angle β may be in the range from 20° to 180°, e.g. in the range from 45° to 180°. Here, the angle α is 250° ± 5° and the angle β is 110° ± 5°.

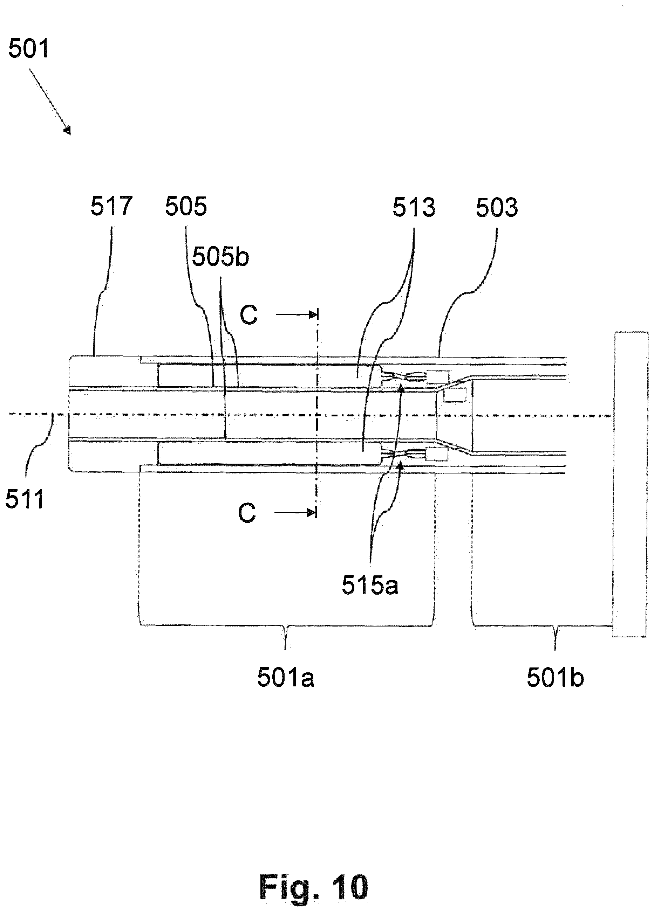

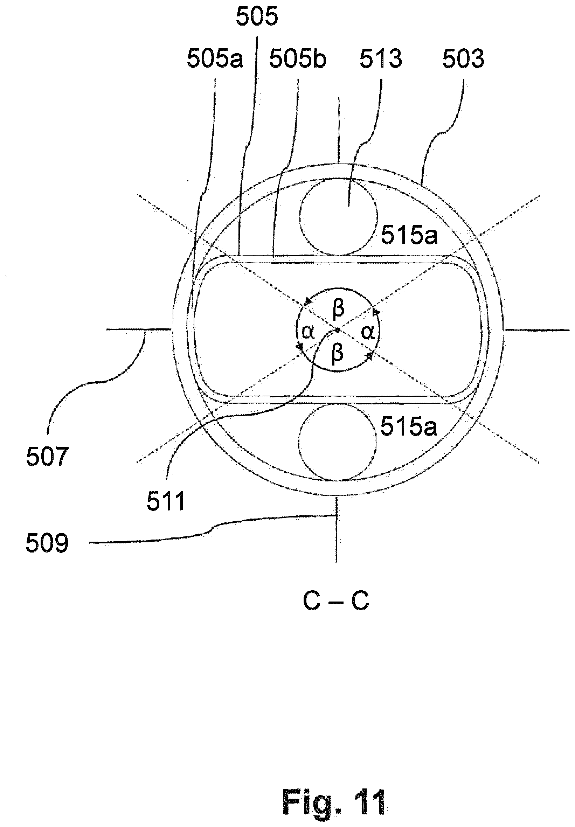

Fig. 10 is a longitudinal cross-section view through the second plane of a fifth surgical tube assembly 501. Fig. 11 shows the cross-section C-C of Fig. 10.

The fifth surgical tube assembly 501 differs from the fourth surgical tube assembly 401 in the plug 517, which closes the first space 515a. The plug 517 corresponds to the plug 417 of the fourth surgical tube assembly 401. However, here, the plug 517 is plane-symmetrical not only to the second plane 509 but also to another plane, i.e. the first plane 507.

Further, in the first section 501a, there are two second circumferential portions 505b on opposite sides of a plane, i.e. the first plane 507, longitudinally intersecting the outer tube 503, and two first spaces 515a. In some variants, as shown here, each of the two first spaces 515a is configured to accommodate, or accommodates, a sensor element 513. In other variants, only one of the two first spaces 515a is configured to accommodate, or accommodates, a sensor element 513, while the other first space 515a is configured to accommodate, or accommodates, a different component (e.g. accommodates a light source). In other words, the first section 501a is plane-symmetrical not only to the second plane 509 but also to the first plane 507.

The first circumferential portions 505a, which are circumferentially distanced from each other, extend circumferentially in total 180° and the second circumferential portions, which are circumferentially distanced from each other, extend circumferentially in total 180° about the centre line 511 of the outer tube 503.

The first circumferential portion 505a is divided into multiple first circumferential portions 505a, which are circumferentially spaced apart from each other, and the second circumferential portion 505b is divided into multiple second circumferential portions 505b, which are circumferentially distanced from each other. Here, the number, e.g. two, of first circumferential portions 505a equals the number, e.g. two, of second circumferential portions 505b. The first circumferential portions 505a each extend circumferentially an angle α about the centre line 511 of the outer tube 503 and the second circumferential portions 505b each extend circumferentially a second angle β about the centre line 511 of the outer tube 503. The total sum of the angles α may be in the range from 320° to 1°, e.g. in the range from 180° to 1°. The total sum of the angles β may be in the range from 40° to 359°, e.g. in the range from 180° to 359°. Here, the total sum of the angles a is 140° ± 5° and the total sum of the angles β is 220° ± 5°.

Of course, in case there are multiple second circumferential portions 505b and, therefore, multiple first spaces 515a, a sensor element 513 can be provided in each of the multiple first spaces 515a.

As has become apparent from the above description of exemplary embodiments, the surgical tube assembly presented herein can efficiently be manufactured to include one or more sensor elements or any other components between an inner and an outer tube.

## Claims

1. A surgical tube assembly (101-501) comprising:
an outer tube (103-503); and
an inner tube (105-505), wherein the inner tube (105-505) has a uniform wall thickness and wherein, in a first section (101a-501a) of the surgical tube assembly (101-501), a first circumferential portion (105a-505a) of the inner tube (105-505) is supported at the outer tube (103-503) and at least one first space (115a-515a) configured to accommodate a sensor element (113-513) is defined between at least one second circumferential portion (105b-505b) of the inner tube (105-505) and the outer tube (103-503).

2. The surgical tube assembly (101-501) according to claim 1, wherein,
in the first section (101a-501a), the second circumferential portion (105b-505b) is flat.

3. The surgical tube assembly (101-501) according to claim 1 or 2, wherein,
in the first section (101a-501a), a transition from an outer surface of the second circumferential portion (105b-505b) to an outer surface of the first circumferential portion (105a-505a) is curved.

4. The surgical tube assembly (101-501) according to any one of claims 1 to 3, wherein,
in the first section (101a-501a), an outer surface of the first circumferential portion (105a-505a) abuts an inner surface of the outer tube (103-503).

5. The surgical tube assembly (501) according to any one of claims 1 to 4, wherein,
in the first section (501a), there are two second circumferential portions (505b) on opposite sides of a plane (507) longitudinally intersecting the outer tube (503), and two first spaces (515a).

6. The surgical tube assembly (101-401) according to any one of claims 1 to 5, wherein,
in the first section (101a-401a), the inner tube (105-405) has a D-shaped cross-section.

7. The surgical tube assembly (101-501) according to any one of claims 1 to 6, wherein,
in a cross-section of the first section (101a-501a), an area occupied by a wall of the inner tube (105-505) is less than an area enclosed by a wall of the inner tube (105-505).

8. The surgical tube assembly (101-501) according to any one of claims 1 to 7, wherein,
in a second section (101b-501b) of the surgical tube assembly (101-501) that is spaced apart from the first section (101a-501a) along an extension of the surgical tube assembly (101-501), the first circumferential portion (105a-505a) is larger and the second circumferential portion (105b-505b) is smaller than in the first section (101a-501a).

9. The surgical tube assembly (101-501) according to claim 8, wherein
the second space (115b-515b) is open at a first end of the surgical tube assembly (101-501).

10. The surgical tube assembly (101-501) according to any one of claims 1 to 9, further comprising:
the sensor element (113-513), which is located in the first space (101a-501a).

11. The surgical tube assembly (101-501) according to claim 10, wherein
the sensor element (113-513) is fixed to the second circumferential portion (105b-505b).

12. The surgical tube assembly (101-501) according to any one of claims 1 to 11,
wherein
the first space (115a-515a) is arranged in a region of a second end of the surgical tube assembly (101-501) and closed at the second end.

13. The surgical tube assembly (101-501) according to claim 12, further comprising:
a plug (117-517) closing the first space (115a-515a) at the second end.

14. A surgical instrument (1) configured as a surgical suction instrument comprising the surgical tube assembly (101-501) according to any one of claims 1 to 13, wherein
the inner tube (105-505) defines a suction channel.

15. A surgical instrument (1) configured as a surgical endoscope comprising the surgical tube assembly (101-501) according to any one of claims 1 to 13, wherein
the inner tube (105-505) houses one or more endoscopic components.

## Amended claims

### Amended claims in accordance with Rule 137(2) EPC.

1. A surgical tube assembly (101-501) comprising:
an outer tube (103-503); and
an inner tube (105-505), wherein, in a first section (101a-501a) of the surgical tube assembly (101-501), a first circumferential portion (105a-505a) of the inner tube (105-505) is supported at the outer tube (103-503);
**characterized in that**
the inner tube (105-505) has a uniform wall thickness; and
at least one first space (115a-515a) configured to accommodate a sensor element (113-513) is defined between at least one second circumferential portion (105b-505b) of the inner tube (105-505) and the outer tube (103-503).

2. The surgical tube assembly (101-501) according to claim 1, wherein,
in the first section (101a-501a), the second circumferential portion (105b-505b) is flat.

3. The surgical tube assembly (101-501) according to claim 1 or 2, wherein,
in the first section (101a-501a), a transition from an outer surface of the second circumferential portion (105b-505b) to an outer surface of the first circumferential portion (105a-505a) is curved.

4. The surgical tube assembly (101-501) according to any one of claims 1 to 3, wherein,
in the first section (101a-501a), an outer surface of the first circumferential portion (105a-505a) abuts an inner surface of the outer tube (103-503).

5. The surgical tube assembly (501) according to any one of claims 1 to 4, wherein,
in the first section (501a), there are two second circumferential portions (505b) on opposite sides of a plane (507) longitudinally intersecting the outer tube (503), and two first spaces (515a).

6. The surgical tube assembly (101-401) according to any one of claims 1 to 5, wherein,
in the first section (101a-401a), the inner tube (105-405) has a D-shaped cross-section.

7. The surgical tube assembly (101-501) according to any one of claims 1 to 6, wherein,
in a cross-section of the first section (101a-501a), an area occupied by a wall of the inner tube (105-505) is less than an area enclosed by a wall of the inner tube (105-505).

8. The surgical tube assembly (101-501) according to any one of claims 1 to 7, wherein,
in a second section (101b-501b) of the surgical tube assembly (101-501) that is spaced apart from the first section (101a-501a) along an extension of the surgical tube assembly (101-501), the first circumferential portion (105a-505a) is larger and the second circumferential portion (105b-505b) is smaller than in the first section (101a-501a).

9. The surgical tube assembly (101-501) according to claim 8, wherein
the second space (115b-515b) is open at a first end of the surgical tube assembly (101-501).

10. The surgical tube assembly (101-501) according to any one of claims 1 to 9, further comprising:
the sensor element (113-513), which is located in the first space (101a-501a).

11. The surgical tube assembly (101-501) according to claim 10, wherein
the sensor element (113-513) is fixed to the second circumferential portion (105b-505b).

12. The surgical tube assembly (101-501) according to any one of claims 1 to 11, wherein
the first space (115a-515a) is arranged in a region of a second end of the surgical tube assembly (101-501) and closed at the second end.

13. The surgical tube assembly (101-501) according to claim 12, further comprising:
a plug (117-517) closing the first space (115a-515a) at the second end.

14. A surgical instrument (1) configured as a surgical suction instrument comprising the surgical tube assembly (101-501) according to any one of claims 1 to 13, wherein
the inner tube (105-505) defines a suction channel.

15. A surgical instrument (1) configured as a surgical endoscope comprising the surgical tube assembly (101-501) according to any one of claims 1 to 13, wherein
the inner tube (105-505) houses one or more endoscopic components.
